# EUROPEAN PATENT APPLICATION

(11) **EP 3 954 284 A1**
(43) Date of publication of application: **16.02.2022**
(21) Application number: 20787919.8
(22) Date of filing: 03.04.2020
(51) Int. Cl.: A61B 5/05

(54) **SYSTEM FOR INVESTIGATING BIOLOGICAL OBJECTS**

(30) Priority: 11.04.2019 RU 2019111042
(71) Applicant: Obshchestvo S Ogranichennoj Otvetstvennost'yu "Laboratoriya Imeni Vladimira Anatol'evicha Burceva", St. Petersburg, 191023 (RU)
(72) Inventor: BUR EV, Vladimir Anatol'evich, St.Petersburg, 191036 (RU); BOL'SHAKOV, Evgenij Pavlovich, St.Petersburg, pos. Pontonnyj, 196643 (RU); KALININ, Nikolaj Valentnovich, St. Petersburg, 195427 (RU)
(74) Representative: Patpol Kancelaria Patentowa Sp. z o.o.
(86) International application number: PCT/RU2020/000185
(87) International publication number: WO 2020/209758

(57) **Abstract**

The invention relates to the field of biology and medicine and can be applied in examination of live biological objects, such as cells. A system for examination of biological objects, comprising a multirange light source, connected through a technological chamber and a bellows unit with a diagnostic chamber, to which a sensor of transmitted radiation and a sub-microscope are connected, wherein the multirange light source comprises a high-voltage pulse generator with two double forming artificial lines, at least two peakers, at least one transmitting coaxial line, conical matching line, capillary electric discharge load; the technological chamber comprises a differential pumping unit with a pulsed gas valve; the diagnostic chamber comprises a target unit removably receiving a changeable sample of a biological object therein; the bellows unit is constructed with a possibility to focus radiation from the multirange light source on the target unit of the diagnostic chamber, wherein the sensor of transmitted radiation comprises a detector of radiation located relative to the target unit in a way enabling to register incident radiation and/or radiation transmitted through the target unit; and the sub-microscope comprises an illuminating and imaging objectives, a detector of radiation and a holder for the changeable sample of the biological object. A technical result of the claimed invention is improvement of resolution of the biological object image obtained, due to the possibility of changing of the wavelength at which the sub-microscope will have the maximum resolution.

## Description

The invention relates to the field of biology and medicine and can be applied for examination of live biological objects, such as cells.

Among modern threats in Life Sciences, including biology and medicine, there are high mortality from oncological diseases, not sufficient effectiveness of existing measures for early prevention and treatment of such diseases, ambiguous understanding of mechanisms of degeneration of healthy cells into cancer cells, non-effective rehabilitation system, critical retardation of modern scientific and research facilities for fundamental research and industrial basis for biotechnologies, etc.

The present invention provides a real-time examination of changes of live cells and cancer affected cells in an organism (a nutrient medium), additionally, depending on the effect of multirange electromagnetic radiation on them. This will facilitate early and unambiguous detection and treatment of a malignant disease, and, above all, search of activation paths for natural self-destruction pathways of cancer affected cells as foreign ones. Additionally, it is proposed to use both known water and carbon windows (2-4 and 4.5-5 nm, respectively), and to do theoretical search for new spectral windows to enhance experimental diagnostic capabilities of the system proposed herein.

Development of new visualization methods in vivo (in an organism) of certain cell groups for cell regeneration medicine and other fields of medicine is becoming a prospective line of modern biomedical research [1]. Among presently applied and newly developed methods of cell structure visualization, a photon-based method using principles of optical coherence tomography (X-ray nanotomography) [2, 3, 4] presents a distinctive advantage due to its high informative value and absence of any necessity to preliminarily prepare biological objects, absence of ionizing radiation (besides special cases of purposeful damage of cancer cells). In the standard method of optical coherence tomography (OCT) three-dimensional information about the object is obtained through observation and registration of optical images based on registration of a signal generated in an object of the study inside different layers with high depth selectivity and high spatial resolution.

Papers [1-4] demonstrate advances in development of direct imaging by X-ray microscopy and its different applications including three-dimensional biological tomography, dynamic processes in magnetic nanostructures, chemical spatial studies, technological applications related to solar cells and batteries, archaeological materials, historical applications. Among these advances there are high-quality images of a single live cell acquired with XM-1 microscope with synchrotron Advanced Light Source at Berkley Lab [2]. A setup like XM-ALS is a quite expensive, sophisticated piece of equipment and is not available to a wide range of potential users.

For the first time a high-brightness EUV-SX (extreme-ultraviolet - soft X-ray) capillary discharge laser with the quantum transition at 46.9 nm of neon-like argon was created at Colorado State University, US, by Professor J. Rocca and his colleagues [5]. This work has been repeated in Italy, Japan, Israel, Czech Republic, and Russia. J. Rocca successfully applied his laser at 46.9 nm for visualization of carbon tubes acquiring their image with a single shot [6]. In this paper it was suggested to obtain an image with a spatial resolution of an order of a wavelength. This suggestion was validated in paper [2], where 12 nm resolution was obtained at visualization of a cell in a nutrient medium using a shorter wavelength (synchrotron) light source. But attempts to make a capillary discharge laser using active mediums based on multicharged ion plasma with shorter wavelengths, due to sharply rising requirements to the required pumping power, would not lead to the success and were mainly abandoned.

Nevertheless, development of shortwave electrical discharge non-laser light sources enabled not only to visualize dry nanometer objects, but also experiments on the effect of such radiation on biological materials and structures, including wet ones. Thus, in papers [7-9] biological materials (DNA, fats, proteins, carbohydrates, chromatin, nucleosomes) were examined in the carbon window of the spectrum. This allowed to study not only superficial, but also deep interaction processes and their diagnostics at a distance up to 30 µm.

Czech researchers have contributed to development of EUV-SX lasers for microscopy. They not only advanced in terms of shorter wavelengths of radiation in electric discharge lasers, and, consequently, in terms of a higher resolution, but, having studied neon-like nitrogen radiation with the wavelength of 2.88 nm being within the water window, they also performed soft X-ray microscopy using an ellipsoidal mirror as an illuminating objective. Preliminary results on visualization of a testing grid with near 110 nm resolution have been obtained [10].

At D.V. Efremov Institute of Electrophysical Apparatus two modifications of "Extreme" setup based on a low-inductive extended z-discharge with closely spaced electrodes have been suggested [11-14]. Compared to known electric discharge EUV lasers, including [5], where an additional microsecond discharge is used for working gas preionization, a sliding -100 ns low-inductive extended avalanche z-discharge have been applied for the first time. Studies with argon pressure changing from 0.001 to 1000 Torr revealed electromagnetic radiation with different sensors in various parts of the spectrum for the first time [11]. Division into bands was quite conventional, but nevertheless it allowed to call this light source a multirange light source.

The sliding avalanche discharge itself, as studies with CCD detector on first modifications of "Extreme" setup have demonstrated, is a glowing circular plasmic formation and not accompanied by any shortwave radiation. But once its front reaches the exit electrode, the discharge transits into a high-voltage longitudinal discharge stage with generation of EUV-SX radiation. A flash of this radiation of several nanoseconds occurring in a very narrow pressure range from 0.2 to 0.4 Torr is of the resonance nature and, probably, is caused by deceleration of runaway electrons on the gas. This process is interrupted by the next stage, development of a longitudinal high-current pinching discharge and pumping of the active medium by thermal electrons.

At pressures less than 0.1 Torr long-pulse generation of hard rays with photon energy 15-25 keV is observed, what is caused by deceleration of runaway electrons on discharge tube walls, as it does not depend on residual gas pressure. This deceleration emission is a slightly divergent beam (less than 10⁻² rad), what can be an effect of its collimation over the long tube Ø5 mm x L100 mm.

As a prototype of the claimed invention, the technical solution described in [6] is chosen. The known setup comprises an electric discharge capillary type laser and a sub-microscope (fractions of µm), consisting of an illuminating Schwarzschild objective with a multilayer Sc-Si-film coating of mirror surfaces, and an imaging objective in the form of a zone plate, as well as CCD cameras.

However, the prototype has drawbacks such as limited functionality due to possibility to work only in one range of wavelengths, as it is used for observation of an object, and the effect on the object is not studied.

The present invention provides solution to a problem of creation of cost-effective practical equipment for fundamental research of the photon-based effect of multirange radiation on nanobiological objects, including cells, and visualization of results of this effect, also using photon-based sub-microscopy. The result of the solution of this problem is planned to be used in clinical equipment for early diagnostics of cancer diseases at the cellular level, and namely for a study of definition of radiation doses in different spectral regions for such processes as affection, changing and division/proliferation. Possible resonance effects of activation of self-destruction pathways of cancer cells as foreign ones are of a particular interest.

A technical result of the claimed invention is improvement of resolution of the biological object image obtained, due to the possibility of changing of the wavelength at which the sub-microscope will have the maximum resolution. Simultaneously, enhancing of functionality is achieved due to creation of new functions, such as visualization and study of behavior of a biological object under the effect of radiation at different wavelengths.

The claimed technical result is achieved due to that the system for examination of biological objects comprises a multirange light source, connected via a technological chamber and a bellows unit with a diagnostic chamber, to which a sensor of transmitted radiation and a sub-microscope are connected. Additionally, the multirange light source comprises a high-voltage pulse generator with two double forming artificial lines, at least two peakers, at least one transmitting coaxial line, conical matching line, capillary electric discharge load. The technological chamber comprises a differential pumping unit with a pulsed gas valve. The diagnostic chamber comprises a target unit removably receiving a changeable sample of a biological object therein. The bellows unit is constructed with a possibility to focus radiation from the multirange light source onto the target unit of the diagnostic chamber. Additionally, the sensor of transmitted radiation comprises a detector of radiation and is located relative to the target unit in a way enabling to register incident radiation and/or radiation transmitted through the target unit. The sub-microscope comprises an illuminating and imaging objectives, a detector of radiation, and a holder for the changeable sample of a biological object.

In one possible embodiment of the main technical solution, accumulating double forming artificial lines are used.

In another possible embodiment of the main technical solution, multi-link double forming artificial lines are used.

In another possible embodiment of the main technical solution, transmitting coaxial lines are divided by the peaker.

In another possible embodiment of the main technical solution, an annular gas peaker is used as a peaker.

In another possible embodiment of the main technical solution, a cylindrical gas peaker is used as a peaker.

In another possible embodiment of the main technical solution, the target unit is a diaphragm and consists of a holder stand for the changeable sample of a biological object.

In another possible embodiment of the main technical solution, the illuminating and imaging objectives are Schwarzschild objectives with a multilayer coating.

Accordingly, the claimed technical result is reached due to the combination of critical features of the invention. To enhance experimental possibilities of the setup during fundamental studies of the effect of radiation on nano-objects, including biological objects (healthy and cancer cells), a low-inductive discharge with preionization of the gas by a sliding avalanche discharge was proposed and applied. This enabled to implement different mechanisms of creation of multicharged ion plasma, including (pinching, non-pinching - beam and combined) mechanisms of pumping and generation of radiation. This enables to get slightly divergent spontaneous, low-coherent and coherent radiation in different spectral ranges, including ranges of extreme ultraviolet (EUV), soft X-rays (SX) and hard X-rays (HX) by a simple change of the initial pressure and type of the gas in the capillary discharge, and, consequently, to get a necessary wavelength. To enable the effect of radiation on biological objects, the technological vacuum chamber is introduced into the system, the chamber is located near the exit of a capillary tube. The pulsed electrical valve located inside the chamber closes the inlet opening of the differential pumping unit with its leaf and opens it before the shot (shot group) to let radiation pass into the beam transfer path, to targets of the target unit located in the diagnostic chamber. Thus, due to the control of number of radiation pulses, pressure, time of the effect, it is possible to define behavior of a certain biological object, what is aimed to get the resonance and, as a result, to reveal self-destruction pathways of malignant cells. Moreover, together with the Schwarzschild illuminating objective, like in the prototype, the use of the imaging objective of the same type instead of the zone plate allowed to unify these microscope systems and apply them for visualization of biological objects both in transmitted and reflected light. This gave additional benefits in experiments on visualization, as well as in the use of schemes with diffusion of illuminating radiation.

Moreover, the use of accumulating double forming artificial lines allowed to avoid the use of special charging devices, as soon as accumulation is performed from a standard source. Using of multi-link double forming artificial lines, including, for example, 4-link, enabled to form pulses whose shape is close to rectangular. The use of the annular gas peaker as a peaker, for instance, 2-section, is explained by the fact that self-breakdown of one of its gaps leads to deformation of the field in the second gap and its breakdown, what results in additional peaking of pulses. The use of the cylindrical gas peaker as a peaker with LC structural parts allows to obtain additional capabilities of the peaker. The use of Schwarzschild objectives as illuminating and imaging objectives leads to unification of objectives and additional increase in measurement accuracy.

The character of the present invention and its embodiments are further disclosed in the following description with reference to accompanying drawings.
Fig. 1 illustrates the flow-chart of the claimed system.
Fig. 2 illustrates position of elements of the system.

In the figures the following is indicated:
1 - multirange light source (MRLS);
2 - technological chamber (TC);
3 - bellows unit (BU);
4 - diagnostic chamber (DC);
5 - sensor of transmitted radiation (STR);
6 - sub-microscope (SM);
7 - high-voltage pulse generator (HVPG);
8 - annular gas peaker (AGP);
9 - cylindrical gas peaker (CGP);
10 - transmitting coaxial line (TCL);
11 - transmitting coaxial line (TCL);
12 - conical matching line (CML);
13 - capillary electric discharge load (CEDL);
14 - power system (PW);
15 - gas supply system (GSS);
16 - differential pumping unit (DPU);
17 - pumping system (PS) of the vacuum diagnostic chamber;
18 - pumping system (PS) of the sub-microscope vacuum chamber;
19 - pressure measuring system (PMS) of the residual gas in the technological chamber;
20 - pressure measuring system (PMS) of the residual gas in the chamber of the sub-microscope;
21 - gate valve (GV);
22 - information control system (ICS).

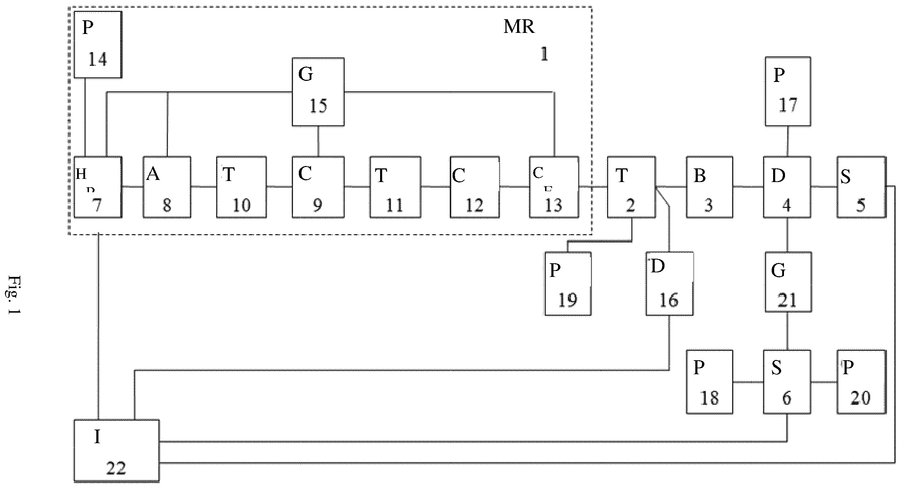

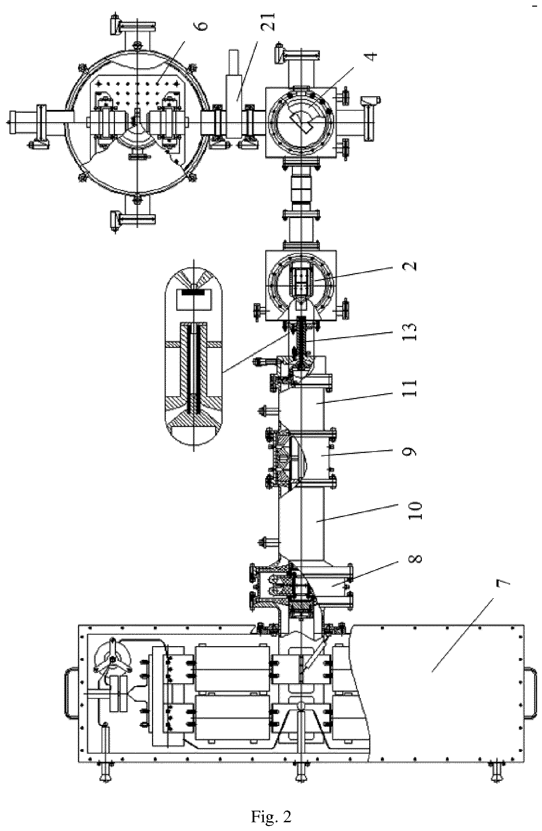

The system for examination of biological objects (Figs. 1, 2) comprises a multirange light source 1 (MRLS), connected through a technological chamber 2 (TC) and a bellows unit 3 (BU) with a diagnostic chamber 4 (DC), to which a sensor 5 of transmitted radiation (STR) and a sub-microscope 6 (SM) are connected.

MRLS 1 comprises a series-connected and located in the same body high-voltage pulse generator 7 (HVPG) for powering of a low-induction z-discharge with two double forming artificial lines (DFL) (not shown in the drawing), at least two peakers, for example, an annular gas peaker 8 (AGP) and/or a cylindrical gas peaker 9 (CGP), at least one transmitting coaxial line (TCL) 10 and 11 (in the given implementation TCL are divided by CGP 9), a conical matching line 12 (CML), and a capillary electric discharge load 13 (CEDL). Additionally, MRLS 1 comprises power system 14 (PW) of DFL (not shown in the drawing), an accumulating capacitor (not shown in the drawing) of HVPG 7 and a gas supply system 15 (GSS) of linear commutation switches of HVPG 7, AGP 8, CGP 9, and CEDL 13.

DFLs (not shown in the drawing), due to the use of the low-leak paper-oil dielectric, can be made accumulating, i.e., continuously charged with high-voltage rectifiers from the mains supply. DFLs (not shown in the drawing) also can be constructed as multi-link DFLs for forming a pulse whose shape is majorly close to rectangular. DFLs (not shown in the drawing) can be assembled according to a folded chain from rolled capacitor sections and connected in parallel into a "butterfly" scheme with a current collector unit. DFLs (not shown in the drawing) are triggered by linear gas commutation switches (not shown in the drawing) with deformation of the electrical field. For their triggering, a thyratron RC-generator is used, which forms pulses, and dividing ceramic disk capacitors (not shown in the drawing) and a ferrite ring cable-type transformer (not shown in the drawing) with stepping-up of the input voltage at a rate 3:1. All this is put into a tank with transformer oil.

Further, high-voltage pulses generated by DFLs (not shown in the drawing) go through peaking of the rising edge by AGP 8 with self-breakdown of the first gap and deformation of the field in the second gap, and CGP 9 with L-C breakdown of other gaps. Between AGP 8 and CGP 9 there are sections of TCL 10 and 11 with 50% water-glycerin dielectric. Moreover, TCL can be made both of one piece and of several separated pieces. The exit end of TCL 11 is connected to CEDL 13 through CML 12.

CEDL 13, through a sliding seal (not shown in the drawing), is connected to TC 2, which comprises a differential pumping unit 16 (DPU) installed at the opposite side to the joint with CEDL 13, the unit being a combination of diaphragms installed at a distance from each other and interfering gas passage, and a pulsed gas valve (not shown in the drawing) protecting the radiation transfer path from inleakage of the gas from the capillary, as gas inleakage limits the number of pulse repetitions. DPU 16 with pulsed gas valve provides modeling of the frequency mode of the pulse sequence and generation of a pulse series from MRLS 1.

The proposed MRLS 1 enables to generate the following wavelength ranges (division into bands is conventional):
1. Hard X-rays, photon energy is 15 to 25 keV (wavelength less than 0.1 nm) - argon pressure 0.01 to 0.2 Torr.
2. Soft X-rays, photon energy is from near tens of eV to units of keV (wavelength from fractions of nm to units of nm) - argon pressure 0.2 to 0.4 Torr.
3. Extreme ultraviolet radiation, photon energy is from several tens of eV to several hundreds of eV (wavelength from several nm to several tens of nm) - argon pressure 0.4 to 0.6 Torr.
4. Visible radiation, wavelength of 180-500 nm - argon pressure 20 to 200 Torr.
5. Infrared radiation, wavelength of 600-1000 nm - argon pressure 300 to 750 Torr.

DC 4 comprises a target unit (not shown in the drawing) removably receiving a changeable sample of a biological object (not shown in the drawing) therein. STR 5 is installed at the exit of DC 4, STR is located relative to the target unit (not shown in the drawing) in a way enabling to register incident radiation and/or radiation transmitted through it and contains a detector of radiation with calibrated semiconductor photodiodes for registration of radial distribution of both incident radiation and radiation transmitted through the changeable sample. DC 4 can also be equipped with compact spectrometers (not shown in the drawing).

The target unit (not shown in the drawing) is a diaphragm and consists of a holder stand with micrometer adjusting screws for mounting and fixing interchangeable samples of biological objects and of the target simulator (for adjustment) in the form of a square plate with a central opening of a diameter providing sufficiently uniform distribution of the radiation intensity over the radius of the opening, and the plate being inserted in the vertical grooves of the unit. During experiments with biological objects the target simulator is replaced by a sample of a biological object, which is, for example, a portable sealed cartridge consisting of two air-tight films transparent for the radiation, between them there is a biostructure in a nutrient medium illuminated through the central opening in the gripping plates.

BU 3 is constructed with a possibility to focus radiation from MRLS 1 onto the target unit (not shown in the drawing) of DC 4. BU 3 is a corrugated metal tube equipped with adjusting means (e.g., bolts) of the tube angle, and it is used for a rough adjustment of the beam on the target unit (not shown in the drawing).

A chamber of SM 6 is mounted on the flange of DC 4. On the upper flange of the chamber of SM 6 a turbomolecular pump (not shown in the drawing) is mounted, with a vacuum volute forepump (not shown in the drawing) connected to it. SM 6 comprises an illuminating and imaging objectives, a detector of radiation, and a holder for the changeable sample of a biological object. Illuminating and imaging objectives can be Schwarzschild objectives. The objectives contain mirrors with multilayer coatings on their surface defining their spectral characteristics. The objectives (or their components) are changeable, depending on the wavelength range of effecting radiation. The illuminating and imaging objectives with focusing elements are mounted in the vacuum chamber of SM 6 on a platform and have adjustment devices. Objects being examined (biological objects samples) are mounted on the holder, also having adjustment devices. The detector of radiation for visualization and registration of images of the targets (the simulator and the sample) can be a CCD camera with a luminophor coating. It is provided to do experiments on effect of radiation on nanoobjects and visualization of results of the effect both in reflected and transmitted light, depending on the location of the detector of radiation and whether a transparent or opaque substrate is used for the changeable sample of a biological object (e.g., of a cartridge).

DC 4 and the chamber SM 6 are equipped with pumping systems (PS) 17 and 18 with pumps. PSs 17 and 18 can be united into one pumping system.

TC 2 and the chamber of SM 6 are equipped with vacuum sensors, which are pressure measurement systems (PMS) 19 and 20, for example, with a Pirani sensor with a two-channel controller.

Between the chamber of SM 6 and DC 4 a gate valve 21 (GV) can be installed, which is a vacuum unit for PS.

The system can be controlled manually or automatically by an information control system 22 (ICS), comprising control units of power, gas supply, pumping, operation, as well as of a unit of processing of obtained images, etc. ICS 22 can be connected with MRLS 1, STR 5, DPU 16, SM 6, as well as with other sensors and digital control systems of the system.

It is proposed that the claimed system be installed in a shielded measuring room with an oscilloscope.

The system implements the following method of examination of biological objects.

Initially, adjustment, centering and focusing of the elements of the system are performed. To do this, in the target unit of DC 4 a target simulator is installed (not shown in the drawing), and visible radiation is directed on it, for example, from an additional light source, e.g. LED (not shown in the drawing) installed on the axis of MRLS 1, for example, from the output of CEDL 13, and, if necessary, the target simulator position (not shown in the drawing) is adjusted by adjusting screws (not shown in the drawing) in the holder of the target unit (not shown in the drawing) to ensure that the radiation comes into its center. Then the target simulator (not shown in the drawing) is carried into the holder of SM 6, and on its place in DC 4 a pivoting mirror (not shown in the drawing) is installed, if necessary, the pivoting mirror's position (not shown in the drawing) is adjusted by adjusting screws (not shown in the drawing) in the holder of the target unit (not shown in the drawing), and the target simulator position (not shown in the drawing) is adjusted in the holder of SM 6 to ensure that the radiation comes into the center of the target simulator (not shown in the drawing). Focusing is performed by spatial movement of objectives of SM 6.

During experiments with biological objects, the target simulator (not shown in the drawing) is replaced by a biological object sample (not shown in the drawing). Then generation of radiation of a required wavelength is performed by MRLS 1, it is then directed onto the examined biological object sample (not shown in the drawing), the radiation reflected or transmitted through the sample is registered by STR 5 and the absorbed radiation dose is defined. The examined biological object sample (not shown in the drawing) is then carried into the chamber of SM 6 and fixed in the holder between the objectives. In DC 4 the pivoting mirror (not shown in the drawing) is installed. Further, the wavelength of MRLS 1 radiation is changed to the optimal one to achieve the best spatial resolution, it is then used to illuminate the sample, visualized by objectives of SM 6 and registered by the CCD camera. If necessary, the radiation dose is corrected. Based on the acquired data, conclusions about changes occurred in the biological object, depending on parameters of radiation used for the effect on the object, are made.

Example of application of the claimed system.

Initially, the leaf of the pulsed valve closes the entrance opening of 1 to 2 mm diameter (changeable) of DPU 16 very tightly, i.e., the valve is closed. At this moment, the path should be pumped out (ES 17 and 18) up to a high pressure 10⁻⁴ Torr, measuring the pressure with a Pirani sensor with a controller (PMS 19, 20). Pump out CEDL 13 and TC 2 up to the forevacuum limit of 10⁻² Torr measuring the pressure.

Then fill the gas into TC 2 and, consequently, into CEDL 13 up to a required pressure in the range of 10⁻²-1000 Torr, registering the value with an autonomous vacuum Pirani sensor.

Fill linear commutation switches at the entrance of DFL (not shown in the drawing) with argon up to a pressure -3.5 atm. depending on the voltage of the discharge of DFL of 60-90 kV, measure the pressure with an indicating pressure gage.

Fill peakers 8 and 9 with argon up to a pressure ~1 atm. Charge DFL (not shown in the drawing) up to the predefined working value by readouts of the indicator (e.g., indicator board) of the source SL-40 kV-150 Wt, and the thyratron generator on the device TPI1-10 kA/50 up to the optimal value by readouts of thyratron generator power source (IP-40 kV-15Wt).

Trigger the pulsed valve by a synchronizer in ICS 22, while releasing the entrance opening of DPU 16 for passage of the radiation into the path of transfer to nanoobjects.

In 5 seconds launch the generator of drive pulses. Close the entrance opening of DPU 16 to avoid excessive input of the gas into the path (single pulse mode).

The gas filling the unit gradually fills cells of DPU 16, and unless it has not virtually come into the path, a train pulse operation mode can be performed with a limited number of pulses in a train with a repetition frequency of a fraction of Hz.

In a similar way, fundamental studies of processes of the radiation effect in different spectral ranges, putting the target unit with the changeable biological object sample into DC 4, can be performed.

For visualization of results of the effect, put the biological object sample in the chamber of SM 6, replacing it by the pivoting mirror unit, and switch on the CCD camera to acquire the image of the biological object.

Thus, the claimed system enables not only to visualize biological objects themselves, but also to effect on them with further analysis of results.

### References

1. A.V. Meleshina, E.I. Cherkasova, M.V. Shirmanova et al. Modern Techniques for stem Cells in vivo Imaging (Review). Sovremennye Tehnologii v Medicine [Modern Technologies in Medicine], 2015, vol. 7, No. 4. p. 174-188.
2. David Attwood. Nanotomography comes of age. Nature, 2006, v.442, p.642.
3. Anne Sakdinawat and David Attwood. Nanoscale X-ray imaging. Nature Photonics. 2010, v.4, p. 840-8.
4. Weilun Chao, Bruce D. Harteneck, J. Alexander Liddle, Erik H. Anderson and David T. Attwood. Soft X-ray microscopy at a spatial resolution better than 15 nm. Letters Nature, 2005, v.435, 30 June 2005, p. 1210-1213.
5. J.J. Rocca. Table-top soft X-ray lasers. Review of Scientific Instruments. Review article. 1999, v.70, Nº 10, p. 3799-3825.
6. Fernando Brizuela, Courtney A. Brewer, Przemyslaw Wachulak, Dale H. Martz, Weilun Chao, Erik H. Anderson, David T. Attwood, Alexander V.C Vinogradov, Igor A. Artyukov, Alexander G. Ponomareko, Valeriy V. Kondratenko, Mario C. Marconi, Jorge J. Rocca, Carmen S. Menoni. Single-laser-shot extreme ultraviolet imaging of nanostructures with wavelength resolution. Opt. Letters, 2008, v.33, p.518-520.
I.A. Artyukov, B.R. Benware, A.V. Vinogradov, Yu.S. Kas'yanov, V.V. Kondratenko, K.D. Macchetto, A. Ozols, J.J. Rocca, J.L.A. Chilla. Focusing of the beam of a compact, repetitively pulsed X-ray laser for studying the interaction of radiation with metallic targets and X-ray reflectometry. Kvantovaya Elektronika [Quantum Electronics], 2000, 30, 328.
8. I.A. Artyukov, R.M. Feschenko, A.V. Vinogradov, Ye.A., Ye.A. Bugayev, O.Y. Devizenko, V.V. Kondratenko, Yu.S. Kasyanov, T. Hatano, M. Yamamoto, S.V. Saveliev. Soft X-ray imaging of thick carbon-based materials using the normal incidence multilayer optics. Micron, vol.41, Issue 7, October 2010, p. 722-728.
9. T. Parkman, M.E. Nawaz, M. Nevrkla, M. Vrbova, A. A. Jancarek. Water-window based on nitrogen plasma capillary discharge source. Report on 2015 International Workshop on EUV -ray sources. Dublin, Ireland, Nov. 9-11, 2015.
10. V.A. Burtsev, P.N. Aruev, E.P. Bolshakov, V.V. Zabrodskiy, N.V. Kalinin, V.A. Kubasov, V.I. Chernobrovin. Soft X-ray radiation of low-inductive capillary discharge. Voprosy atmnoi nauki i tehniki. Seriya "Elektrofizicheskaja apparatura". [Problems of nuclear science and technics. Series "Electrophysical Apparatus"]. 2010, No. 5(31), p. 251-264.
11. V.A. Burtsev, V.V. Zabrodskiy, N.V. Kalinin (Physical-Technical Institute of the Russian Academy of Sciences), E.P. Bolshakov (Institute of Electrophysical Apparatus). Electromagnetic radiation sources based on a low-inductive extended z-discharge. Zhurnal Tehnicheskoi Fiziki [The journal of technical physics], vol. 83, No. 2, p. 43-51.
12. V.A. Burtsev, N.V. Kalinin, S.A. Vaganov. Multi-range sources of electromagnetic radiation based on a low-inductive extended z-discharge. American Journal of Modern Physics, 2013, v. 2(3), p. 117-123.
13. Vladimir Burtsev, Nikolay Kalinin, Sergey Vaganov. Low inductive extended z-discharge as a manyrange source of radiations. Journal of Advances in physics.2015, Vol.11, No 2, pp. 3023- 3034.
14. I.A. Artyukov, A.V. Vinogradov, N.L. Popov. On contrast of biological X-ray nanomicroscopy. Kvantovaya Elektronika [Quantum Electronics], 2017, v.47, No.11, p. 1041-1045.

## Claims

1. A system for examination of biological objects, comprising a multirange light source, connected through a technological chamber and a bellows unit with a diagnostic chamber, to which a sensor of transmitted radiation and a sub-microscope are connected, wherein the multirange light source comprises a high-voltage pulse generator with two double forming artificial lines, at least two peakers, at least one transmitting coaxial line, conical matching line, capillary electric discharge load; the technological chamber comprises a differential pumping unit with a pulsed gas valve; the diagnostic chamber comprises a target unit removably receiving a changeable sample of a biological object therein; the bellows unit is constructed with a possibility to focus radiation from the multirange light source on the target unit of the diagnostic chamber, wherein the sensor of transmitted radiation comprises a detector of radiation located relative to the target unit in a way enabling to register incident radiation and/or radiation transmitted through the target unit; and the sub-microscope comprises an illuminating and imaging objectives, a detector of radiation and a holder for the changeable sample of the biological object.

2. The system of claim 1, wherein accumulating double forming artificial lines are used.

3. The system of claim 1, wherein multi-link double forming artificial lines are used.

4. The system of claim 1, wherein transmitting coaxial lines are divided by a peaker.

5. The system of claim 1, wherein an annular gas peaker is used as a peaker.

6. The system of claim 1, wherein a cylindrical gas peaker is used as a peaker.

7. The system of claim 1, wherein the target unit is a diaphragm and consists of a holder stand for the changeable sample of a biological object.

8. The system of claim 1, wherein the illuminating and imaging objectives are Schwarzschild objectives with a multilayer coating.
